# EUROPEAN PATENT SPECIFICATION

(11) **EP 3 760 937 B1**
(45) Date of publication and mention of the grant of the patent: **11.05.2022**
(21) Application number: 19830239.0
(22) Date of filing: 02.07.2019
(51) Int. Cl.: F24F 9/00, F24F 13/28, F24F 11/56, F24F 11/00, B08B 5/02, A61L 2/24, A61L 2/00, F24F 120/10, F24F 110/64, F24F 11/30, F24F 11/52, F24F 8/10

(54) **AIR SHOWER GATE**
LUFTDUSCHENTOR
PORTE DE DOUCHE À AIR

(30) Priority: 04.07.2018 KR 20180077655
(43) Date of publication of application: 06.01.2021
(73) Proprietor: Purium Co., Ltd., Siheung-si, Gyeonggi-do (KR); Nam, Dong Kyu, Seongnam-si, Gyeonggi-do 13645 (KR); Mo, Min Wook, Gwangju, 61682 (KR)
(72) Inventor: NAM, Dong Kyu, Seongnam-si, Gyeonggi-do 13645 (KR)
(74) Representative: M. Zardi & Co S.A.
(86) International application number: PCT/KR2019/007996
(87) International publication number: WO 2020/009397

(56) References cited:
- CN-U- 201 826 713
- JP-A- 2006 010 121
- JP-A- 2008 064 403
- JP-B2- 2 714 133
- KR-A- 20090 004 025
- KR-A- 20100 006 276
- KR-A- 20100 088 524
- KR-A- 20130 076 159
- KR-A- 20180 052 793
- KR-A- 20180 052 794
- KR-B1- 101 549 243

## Description

### [Technical field]

The present invention relates to air shower gates and, more specifically, to an air shower gate installed at an indoor/outdoor entrance/exit so as to remove micro dust, hazardous materials, and odor from people entering/exiting the air shower gate, and configured to monitor the quality of indoor/outdoor air such that, through air purification or ventilation, a comfortable and clean indoor environment can be maintained and managed.

### [BACKGROUND ART]

General air shower devices are used to remove dust from clothes, within an air shower booth provided within a special indoor space.

However, as interest in micro dust has recently increased, several methods have been proposed to improve the quality of indoor air.

In schools, apartments, large buildings, etc., indoor air may be polluted by people entering inside from outside. In particular, the need to prevent contaminants such as dust and dirt from entering the inside is emerging due to a recent increase of micro dust.

Air shower devices in the form of a conventional air shower booth such as Korean Patent No. 10-1549243 are provided indoors and thus are inappropriate to remove contaminants such as dust generated while people are moving to inside from outside.

In addition, because conventional air shower booths are invented to prevent inflow of dust in industrial facilities, a spraying method, such as a spraying intensity, of the conventional air shower booths is not appropriate for general people. In particular, because a user's hair is matted and a garment such as a skirt flutters in the wind, the conventional air shower booths are not appropriate for general people. KR 2010 0088524 A discloses a clean-air maintaining apparatus comprises a main body, a clean sensor, a clean air unit, a sanitary processing unit, a negative electrode, a hand washing unit, and a clean controller.

### [DETAILED DESCRIPTION OF THE INVENTION]

### [TECHNICAL PROBLEM]

Provided is an air shower gate having the advantages that dust and the like gathered on clothes during outdoor activities can be removed, a user can move in/out conveniently without having to open/close a door, inflow of dust into the indoor space can be prevented, indoor air is filtered to perform an air purifying function, outdoor air is filtered and then introduced into the indoor space to thereby perform ventilation, air is adaptively sprayed to a user's body part, and air is sprayed in a manner preferred by each user.

### [TECHNICAL SOLUTION]

According to an aspect of the present invention, there is provided an air shower gate according to claim 1.

The air shower gate further includes a hand sterilizer on the vertical frame, wherein the hand sterilizer operates with a proximity sensor.

A height, shoulder height, and face location range of a user may be measured and used by the controller to control a spray hole.

A lateral side spray hole may be formed to have an inclined spraying angle.

Pre-setting information may be received from a wireless terminal of the user through wireless communication to enable customized spraying.

Air purification and ventilation may be enabled based on information of air qualities measured by the second sensor and the third sensor.

### [ADVANTAGEOUS EFFECTS OF DISCLOSURE]

An air shower gate according to an embodiment of the present invention may remove dust and the like gathered on clothes during outdoor activities and may separate indoor and outdoor spaces by using an air curtain, thereby enabling a user to move in/out conveniently without having to open/close a door. The air shower gate may prevent inflow of dust into the indoor space by using the air curtain, and filter indoor air to perform an air purifying function. The air shower gate may filter outdoor air and then introduce the filtered outdoor air into the indoor space according to sensor information, thereby ventilating the air shower gate. The air shower gate may adaptively spray air to the user's body part according to the sensor information such that the user's hair is not blown. The side ejection angle slopes downward such that garments such as skirts are not blown upward/downward. The air shower gate may receive the user's information from a wireless terminal, such as a smartphone, such that air is ejected in a manner preferred by each user.

### [BRIEF DESCRIPTION OF THE DRAWINGS]

FIG. 1 is a perspective view viewed from a right upper side, according to an embodiment of the present invention.
FIG. 2 is a perspective view viewed from a left lower side, according to an embodiment of the present invention.
FIG. 3 is a front side view showing a usage state, according to an embodiment of the present invention.
FIG. 4 is a front side view showing a usage state, according to an embodiment of the present invention.
FIG. 5 is a lateral side view showing a usage state, according to an embodiment of the present invention.

### [BEST MODE]

The prevent invention will now be described more fully with reference to the accompanying drawings, in which exemplary embodiments are shown. In this process, the size or shape of the components shown in the drawings may be exaggerated for clarity and convenience of description. In addition, terms specifically defined in consideration of the configuration and operation of the present invention are only for describing the embodiments of the present invention, and are not intended to limit the scope of the present invention.

It should be noted that in adding reference numerals to the components of each drawing, the same components have the same reference numerals as possible, even if they are displayed on different drawings. In the description, certain detailed explanations of related well-known configurations or functions are omitted when it is deemed that they may unnecessarily obscure the essence of the present invention.

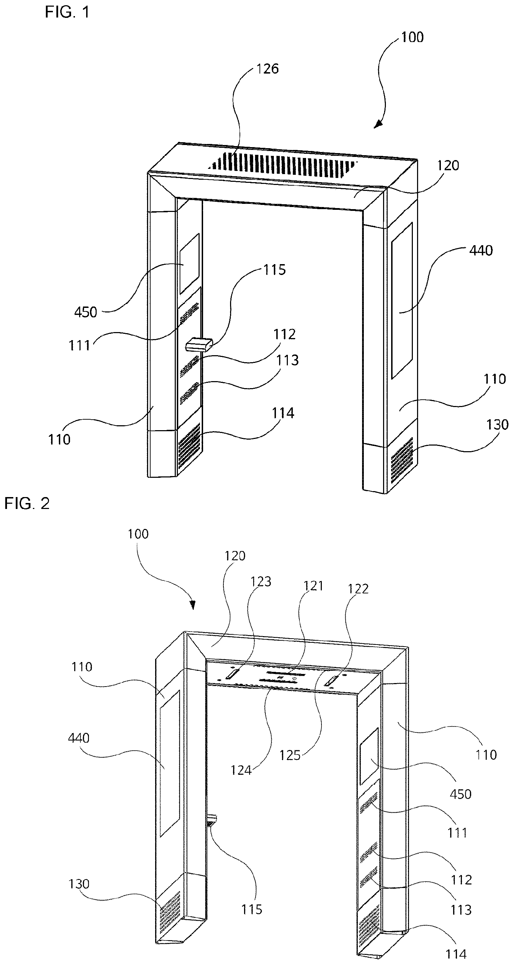

An air shower gate according to an embodiment of the present invention will now be described with reference to FIGS. 1 through 5.

An air shower gate 100 according to the present embodiment is installed at an indoor/outdoor entrance/exit of a building to form an entrance/exit path through which a user may enter or exit.

The air shower gate 100 according to the present embodiment includes a vertical frame 110 and a horizontal frame 120.

The air shower gate 100 according to the present embodiment may be conveniently built even at a conventional entrance/exit to have such a configuration. The vertical frame 110 is provided on either side of an entrance/exit and the horizontal frame 120 is put on the vertical frames 110, and thus the air shower gate 100 may be provided in entrances/exits of various sizes. In this case, a horizontal length adjusting member and a vertical length adjusting member may be combined with a connection unit in order to achieve size adjustment.

The vertical frames 110 are installed adjacent to each other on both left and right sides of the entrances/exit, respectively. A manipulation unit 450, a first lateral side spray hole 111, a second lateral side spray hole 112, a third lateral side spray hole 113, and a polluted air suction hole 114 are formed sequentially from the top on an inner side of each vertical frame 110 that faces the entrances/exit path, and a display 440 and an external surface suction hole 130 are formed on an outer side of the vertical frame 110. According to an embodiment of the present invention, the first, second, and third lateral side spray holes 111, 112, and 113 are configured to independently spray air through fans having discharge holes. A controller may control whether each of the first, second, and third lateral side spray holes 111, 112, and 113 is to spray air, and a spraying intensity of each of the first, second, and third lateral side spray holes 111, 112, and 113. The display 440 displays various pierces of air information, user information, and information necessary for the building on a screen. The display 440 is configured to receive information from an external system and display the received information.

Spraying angles of the first lateral side spray hole 111, the second lateral side spray hole 112, and the third lateral side spray hole 113 are inclined downwards such that a garment such as a skirt of a user does not flutter upwards and downwards.

A hand sterilizer 115 may be further formed on the inner side of each vertical frame 110 that faces the entrances/exit path, and may include a proximity sensor such that the hand sterilizer 115 may operate when a hand of a user approaches.

The horizontal frame 120 is provided over the entrance/exit. On an inner side of the horizontal frame 120 that faces the entrances/exit path, a first upper spray hole 121 is formed at the center, a second upper spray hole 122 is formed on one side of the first upper spray hole 121, a third upper spray hole 123 is formed on the other side of the first upper spray hole 121, a first air curtain 124 is formed at the rear of the first upper spray hole 121 to contact the indoor, a second air curtain 125 is formed in front of the first upper spray hole 121 to contact the outdoor, and an external air suction hole 126 is formed on an external upper surface of the horizontal frame 120. According to an embodiment of the present invention, the first, second, and third upper spray holes 121,122, and 123, and the first and second air curtains 124 and 125 are configured to independently spray air through fans having discharge holes. The controller may control whether each of the first, second, and third upper spray holes 121,122, and 123, and each of the first and second air curtains 124 and 125 is to spray air, and a spraying intensity of each of the first, second, and third upper spray holes 121,122, and 123, and each of the first and second air curtains 124 and 125.

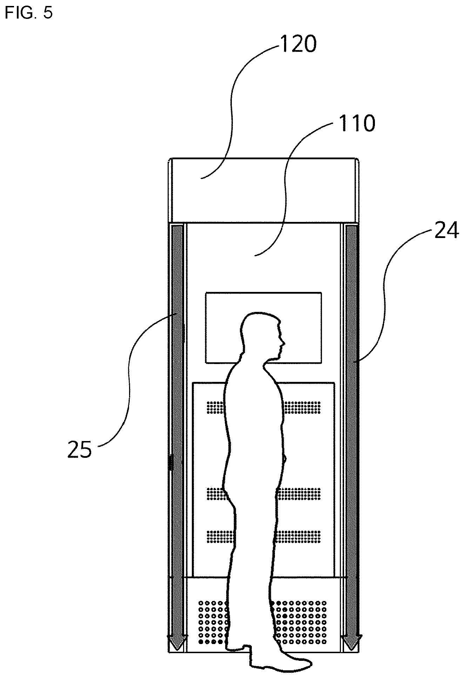

The first and second air curtains 124 and 125 separate indoor and outdoor spaces from the entrance/exit path within the air shower gate 100 when a user has entered the entrance/exit path as shown in FIG. 5, and thus air including dust detached from the user due to air spraying does not flow to the outside of the entrance/exit path and instead is entirely inhaled by the polluted air suction hole 114.

An embodiment of the present invention includes a sensor unit including a first sensor configured to sense a human body existing in a certain area including the entrance/exit path, a second sensor configured to sense the quality of the indoor air, and a third sensor configured to sense the quality of the outdoor air, and each sensor may be located on any of the vertical frame 110 or the horizontal frames 120. According to an embodiment of the present invention, the first sensor is configured to recognize a user through an image sensor, in particular, to recognize each body part of the user, such that the controller may perform control based on body information of the user.

In addition, the external surface suction hole 130 and the external air suction hole 126 include a filter unit that filters sucked air, and thus filter the sucked air, and filtered air moves within each frame and is sprayed from a spray hole.

The controller controls the first lateral side spray hole, the second lateral side spray hole, the third lateral side spray hole, the polluted air suction hole, the first upper spray hole, the second upper spray hole, the third upper spray hole, the first air curtain, and the second air curtain, based on sensing information of the first sensor, sensing information of the second sensor, and sensing information of the third sensor, and is configured to control the spray holes to independently operate or not operate, based on received information, and also adjust a spraying intensity.

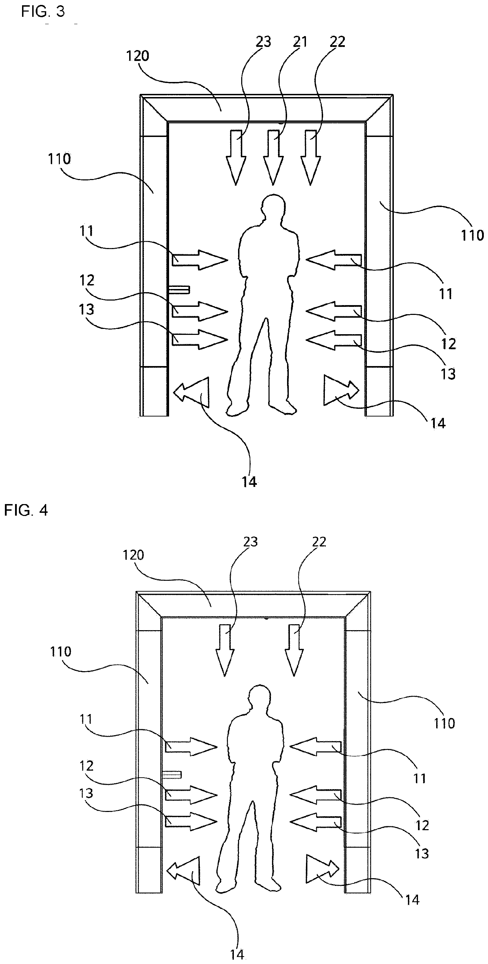

The first sensor measures a height, shoulder height, and face location range of a user who enters and exits the air shower gate 100 and includes the measured height, shoulder height, and face location range in the sensing information, the controller may control a lateral side spray hole having a height less than or equal to the shoulder height to spray air and a lateral side spray hole having a height greater than the shoulder height to not spray air, and the controller may control an upper spray hole within the face location range to not spray air, and may control an upper spray hole out of the face location range to spray air. FIG. 3 illustrates spraying of air to the head of a user, and FIG. 4 illustrates a state where the first spraying hole 121 is controlled to not operate and thus air is not sprayed to the head of a user.

The controller includes a wireless communication transceiver, and the wireless communication transceiver may receive preset information including a spraying time period, a spraying intensity, and the like from a wireless terminal of a user such that the controller may control an operation of a spraying hole.

The second sensor measures a concentration of indoor dust and includes the measured concentration in the sensing information, and accordingly, when the concentration of the indoor dust is higher than a set dust concentration, the controller may control the external surface suction hole and the external air suction hole to suck air and control a spraying hole to spray filtered air.

The third sensor measures a concentration of outdoor dust and a ratio of contaminants within the air for each component and includes a result of the measurement in the sensing information, and thus, when the concentration of outdoor dust and the ratio of contaminants within the air for each component are less than preset values, the controller may control the first air curtain and the second air curtain to not operate so that the indoor air and outdoor air may flow into the indoor space.

### [Explanation of reference numerals]

11: first lateral side sprayed air
12: second lateral side sprayed air
13: third lateral side sprayed air
14: polluted air
21: first upper sprayed air
22: second upper sprayed air
23: third upper sprayed air
24: first air curtain sprayed air
25: second air curtain sprayed air
100: air shower gate
110: vertical frame
111: first side spray hole
112: second side spray hole
113: third side spray hole
114: polluted air suction hole
115: hand sterilizer
120: horizontal frame
121: first upper spray hole
122: second upper spray hole
123: third upper spray hole
124: first air curtain
125: second air curtain
126: external air suction hole
130: external surface suction hole
440: display
450: manipulation unit

## Claims

1. An air shower gate (100) to be provided at an entrance/exit of a building to form an
entrance/exit path through which a user enters/exits, the air shower gate (100) comprising:
two vertical frame (110) to be provided on left and right sides of the entrance/exit, respectively, to be adjacent to each other; and
a horizontal frame (120) to be provided over the entrance/exit,
**characterized by** further comprising: a sensor unit including a first sensor configured to sense a human body existing in a certain area including the entrance/exit path, a second sensor configured to sense the quality of indoor air, and a third sensor configured to sense the quality of outdoor air,
wherein a manipulation unit (450), a first lateral side spray hole, a second lateral side spray hole, a third lateral side spray hole, and a polluted air suction hole (114) are formed sequentially from the top on an inner side of each vertical frame (110) that faces the entrances/exit path,
a display (440) and an external surface suction hole (130) are formed on an outer side of the vertical frame (110),
on an inner side of the horizontal frame (120) that faces the entrances/exit path, a first upper spray hole (121) is formed at the center, a second upper spray hole (122) is formed on one side of the first upper spray hole (121), a third upper spray hole (123) is formed on the other side of the first upper spray hole (121), a first air curtain (124) is formed at the rear of the first upper spray hole (121) facing the indoor, and a second air curtain (125) is formed in front of the first upper spray hole (121) facing the outdoor,
an external air suction hole (126) is formed on an external upper surface of the horizontal frame (120),
each of the external surface suction hole (130) and the external air suction hole (126) includes a filter unit that filters sucked air,
the air shower gate (100) further comprises a controller configured to control the first lateral side spray hole, the second lateral side spray hole, the third lateral side spray hole, the polluted air suction hole (114), the first upper spray hole (121), the second upper spray hole (122), the third upper spray hole (123), the first air curtain (124), and the second air curtain (125), based on sensing information of the first sensor, sensing information of the second sensor, and sensing information of the third sensor,
spraying angles of the first lateral side spray hole, the second lateral side spray hole, and the third lateral side spray hole are inclined downwards,
the first sensor measures a height, shoulder height, and face location range of a user who enters and exits the air shower gate (100) and includes the measured height, shoulder height, and face location range in the sensing information,
the controller is further configured to control any lateral side spray hole having a height less than or equal to the shoulder height to spray air and control any lateral side spray hole having a height greater than the shoulder height to not spray air,
the controller is further configured to control any upper spray hole within the face location range to not spray air, and control any upper spray hole out of the face location range to spray air, and
the first air curtain (124) and the second air curtain (125) spray air from the top to the bottom of the entrance/exit path such that a flow of air inside and outside the entrance/exit path is blocked.

2. The air shower gate (100) of claim 1, wherein
a hand sterilizer (115) is provided within the inner side of the vertical frame (110) that faces the entrances/exit path, and
the hand sterilizer (115) includes a proximity sensor.

3. The air shower gate (100) of claim 1, wherein
the controller includes a wireless communication transceiver,
the wireless communication transceiver receives preset information from a wireless terminal of the user, and
the preset information includes a spraying time period and a spraying intensity.

4. The air shower gate (100) of claim 1, wherein
the second sensor measures a concentration of indoor dust and includes the measured concentration in the sensing information, and
when the concentration of the indoor dust is higher than a set dust concentration, the controller controls the external surface suction hole (130) and the external air suction hole (126) to suck air and controls a spraying hole to spray filtered air.

5. The air shower gate (100) of claim 1, wherein
the third sensor measures a concentration of outdoor dust and a ratio of contaminants within the air for each component and includes a result of the measurement in the sensing information, and
when the concentration of outdoor dust and the ratio of contaminants within the air for each component are less than preset values, the controller controls the first air curtain (124) and the second air curtain (125) to not operate so that the outdoor air may flow into the indoor space.

## Patentansprüche

1. Luftduschentor (100), das an einem Eingang/Ausgang eines Gebäudes vorzusehen ist, um einen Eingangs-/Ausgangsweg zu bilden, durch den ein Benutzer eintritt/austritt, wobei das Luftduschentor (100) umfasst:
zwei vertikale Rahmen (110), die an der linken bzw. der rechten Seite des Eingangs/Ausgangs vorzusehen sind, um aneinander angrenzend zu sein; und
einen horizontalen Rahmen (120), der über dem Eingang/Ausgang vorzusehen ist,
**dadurch gekennzeichnet, dass** es ferner umfasst: eine Sensoreinheit, die einen ersten Sensor, der dazu konfiguriert ist, einen menschlichen Körper abzutasten, der in einem bestimmten Bereich, der den Eingangs-/Ausgangsweg umfasst, vorhanden ist, einen zweiten Sensor, der dazu konfiguriert ist, die Qualität von Innenraumluft abzutasten, und einen dritten Sensor umfasst, der dazu konfiguriert ist, die Qualität von Außenluft abzutasten,
wobei eine Manipulationseinheit (450), ein erstes laterales Seitensprühloch, ein zweites laterales Seitensprühloch, ein drittes laterales Seitensprühloch und ein Ansaugloch (114) für verunreinigte Luft nacheinander von oben an einer Innenseite jedes vertikalen Rahmens (110) ausgebildet sind, die dem Eingangs-/Ausgangsweg zugewandt ist,
eine Anzeige (440) und ein Außenoberflächen-Ansaugloch (130) auf einer Außenseite des vertikalen Rahmens (110) ausgebildet sind,
auf einer Innenseite des horizontalen Rahmens (120), die dem Eingangs-/Ausgangsweg zugewandt ist, ein erstes oberes Sprühloch (121) im Zentrum ausgebildet ist, ein zweites oberes Sprühloch (122) auf einer Seite des ersten oberen Sprühlochs (121) ausgebildet ist, ein drittes oberes Sprühloch (123) auf der anderen Seite des ersten oberen Sprühlochs (121) ausgebildet ist, ein erster Luftvorhang (124) an der Rückseite des ersten oberen Sprühlochs (121) ausgebildet ist, wobei er dem Innenraum zugewandt ist, und ein zweiter Luftvorhang (125) vor dem ersten oberen Sprühloch (121) ausgebildet ist, wobei er dem Außenraum zugewandt ist,
ein Außenluft-Ansaugloch (126) auf einer äußeren oberen Oberfläche des horizontalen Rahmens (120) ausgebildet ist,
sowohl das Außenoberflächen-Ansaugloch (130) als auch das Außenluft-Ansaugloch (126) eine Filtereinheit umfasst, die angesaugte Luft filtert,
das Luftduschentor (100) ferner eine Steuereinrichtung umfasst, die dazu konfiguriert ist, das erste laterale Seitensprühloch, das zweite laterale Seitensprühloch, das dritte laterale Seitensprühloch, das Ansaugloch (114) für verunreinigte Luft, das erste obere Sprühloch (121), das zweite obere Sprühloch (122), das dritte obere Sprühloch (123), den ersten Luftvorhang (124) und den zweiten Luftvorhang (125) basierend auf Abtastinformation des ersten Sensors, Abtastinformation des zweiten Sensors und Abtastinformation des dritten Sensors zu steuern,
Sprühwinkel des ersten lateralen Seitensprühlochs, des zweiten lateralen Seitensprühlochs und des dritten lateralen Seitensprühlochs nach unten geneigt sind,
der erste Sensor eine Höhe, Schulterhöhe und Gesichtspositionsbereich eines Benutzers misst, der das Luftduschentor (100) betritt und verlässt, und die gemessene Höhe, Schulterhöhe und Gesichtspositionsbereich in die Abtastinformation einschließt,
die Steuereinrichtung ferner dazu konfiguriert ist, jedes laterale Seitensprühloch, das eine Höhe aufweist, die geringer oder gleich der Schulterhöhe ist, dazu zu steuern, Luft zu sprühen, und jedes laterale Seitensprühloch, das eine Höhe aufweist, die größer als die Schulterhöhe ist, dazu steuern, keine Luft zu sprühen,
die Steuereinrichtung ferner dazu konfiguriert ist, jedes obere Sprühloch innerhalb des Gesichtspositionsbereichs dazu steuern, keine Luft zu sprühen, und jedes obere Sprühloch außerhalb des Gesichtspositionsbereichs dazu steuern, Luft zu sprühen, und
der erste Luftvorhang (124) und der zweite Luftvorhang (125) Luft von der Oberseite zur Unterseite des Eingangs-/Ausgangswegs sprühen, so dass ein Luftstrom innerhalb und außerhalb des Eingangs-/Ausgangswegs blockiert ist.

2. Luftduschentor (100) nach Anspruch 1, wobei
ein Handsterilisator (115) in der Innenseite des vertikalen Rahmens (110) vorgesehen ist, die dem Eingangs-/Ausgangsweg zugewandt ist, und
der Handsterilisator (115) einen Näherungssensor umfasst.

3. Luftduschentor (100) nach Anspruch 1, wobei
die Steuereinrichtung einen drahtlosen Kommunikationstransceiver umfasst,
der drahtlose Kommunikationstransceiver voreingestellte Information von einem drahtlosen Endgerät des Benutzers empfängt, und
die voreingestellte Information eine Sprühzeitdauer und eine Sprühintensität umfasst.

4. Luftduschentor (100) nach Anspruch 1, wobei
der zweite Sensor eine Konzentration von Innenraumstaub misst und die gemessene Konzentration in die Abtastinformation einschließt, und
wenn die Konzentration des Innenraumstaubs höher ist als eine eingestellte Staubkonzentration, die Steuereinrichtung das Außenoberflächen-Ansaugloch (130) und das Außenluft-Ansaugloch (126) dazu steuert, Luft anzusaugen, und ein Sprühloch dazu steuert, gefilterte Luft zu sprühen.

5. Luftduschentor (100) nach Anspruch 1, wobei
der dritte Sensor eine Konzentration von Außenstaub und ein Verhältnis von Verunreinigungen in der Luft für jede Komponente misst und ein Ergebnis der Messung in die Abtastinformation aufnimmt, und
wenn die Konzentration von Außenstaub und das Verhältnis von Verunreinigungen in der Luft für jede Komponente geringer als voreingestellte Werte sind, die Steuereinrichtung den ersten Luftvorhang (124) und den zweiten Luftvorhang (125) dazu steuert, nicht zu arbeiten, so dass die Außenluft in den Innenraum strömen kann.

## Revendications

1. Porte de douche à air (100) devant être prévue au niveau de l'entrée/sortie d'un bâtiment afin de former une voie d'entrée/sortie à travers laquelle un utilisateur entre/sort, la porte de douche à air (100) comprenant :
deux cadres verticaux (110) devant être prévus sur les côtés droit et gauche de l'entrée/sortie, respectivement, pour être adjacents l'un à l'autre et
un cadre horizontal (120) devant être prévu au-dessus de l'entrée/sortie,
**caractérisé en ce qu'**elle comprend en outre une unité de détection incluant un premier détecteur configuré pour détecter un corps humain présent dans une certaine zone incluant la voie d'entrée/sortie, un deuxième détecteur configuré pour détecter la qualité de l'air intérieur et un troisième détecteur configuré pour détecter la qualité de l'air extérieur,
où une unité de manipulation (450), un premier orifice d'aspersion de côté latéral, un deuxième orifice d'aspersion de côté latéral, un troisième orifice d'aspersion de côté latéral et un orifice d'aspiration de l'air pollué (114) sont formés en séquence à partir du sommet d'un côté interne de chaque cadre vertical (110) qui fait face à la voie d'entrée/sortie,
un affichage (440) et un orifice d'aspiration de la surface externe (130) sont formés sur un côté externe du cadre vertical (110),
sur un côté interne du cadre horizontal (120) qui fait face à la voie d'entrée/sortie, un premier orifice d'aspiration supérieur (121) est formé au centre, un deuxième orifice d'aspersion supérieur (122) est formé d'un côté du premier orifice d'aspersion supérieur (121), un troisième orifice d'aspersion supérieur (123) est formé de l'autre côté du premier orifice d'aspersion supérieur (121), un premier rideau d'air (124) est formé au niveau de l'arrière du premier orifice d'aspersion supérieur (121) faisant face à l'intérieur, et un deuxième rideau d'air (125) est formé devant le premier orifice d'aspersion supérieur (121) faisant face à l'extérieur,
un orifice d'aspiration de l'air extérieur (126) est formé sur une surface supérieure externe du cadre horizontal (120),
chacun parmi l'orifice d'aspiration de la surface externe (130) et l'orifice d'aspiration de l'air extérieur (126) inclut une unité de filtre qui filtre l'air aspiré,
la porte de douche à air (100) comprend en outre un contrôleur configuré pour commander le premier orifice d'aspersion du côté latéral, le deuxième orifice d'aspersion du côté latéral, le troisième orifice d'aspiration du côté latéral, l'orifice d'aspiration de l'air pollué (114), le premier orifice d'aspersion supérieur (121), le deuxième orifice d'aspersion supérieur (122), le troisième orifice d'aspersion supérieur (123), le premier rideau d'air (124) et le deuxième rideau d'air (125), sur la base de l'information détectée par le premier détecteur, de l'information détectée par le deuxième détecteur et de l'information détectée par le troisième détecteur,
les angles d'aspersion du premier orifice d'aspersion du côté latéral, du deuxième orifice d'aspersion du côté latéral et du troisième orifice d'aspersion du côté latéral sont inclinés vers le bas,
le premier détecteur mesure la taille, la hauteur d'épaule et une gamme de localisation du visage d'un utilisateur qui entre et sort de la porte de douche à air (100) et inclut la taille, la hauteur d'épaules et la gamme de localisation du visage mesurées dans l'information détectée,
le contrôleur est en outre configuré pour commander à tout orifice d'aspersion de côté latéral ayant une hauteur inférieure ou égale à la hauteur d'épaule de vaporiser de l'air et commander à tout orifice d'aspersion du côté latéral présentant une hauteur supérieure à la hauteur d'épaules de ne pas vaporiser d'air,
le contrôleur est en outre configuré pour commander à tout orifice d'aspersion supérieur à l'intérieur de la gamme de localisation du visage de ne pas vaporiser d'air et commander à tout orifice d'aspersion supérieur hors de la gamme de localisation du visage de vaporiser de l'air et
le premier rideau d'air (124) et le deuxième rideau d'air (125) vaporisent de l'air depuis le sommet jusqu'en bas de la voie d'entrée/sortie de telle manière qu'une circulation d'air à l'intérieur et à l'extérieur de la voie d'entrée/sortie soit bloquée.

2. Porte de douche à air (100) selon la revendication 1, **caractérisée en ce que** :
un stérilisateur pour les mains (115) est prévu à l'intérieur du côté interne du cadre vertical (110) faisant face à la voie d'entrée/sortie et
le stérilisateur pour les mains (115) inclut un détecteur de proximité.

3. Porte de douche à air (100) selon la revendication 1, **caractérisée en ce que** :
le contrôleur inclut un émetteur-récepteur de communication sans fil,
l'émetteur-récepteur de communication sans fil reçoit de l'information préréglée depuis un terminal sans fil de l'utilisateur et
l'information préréglée inclut une période de temps de vaporisation et une intensité de vaporisation.

4. Porte de douche à air (100) selon la revendication 1, **caractérisée en ce que**
le deuxième détecteur mesure une concentration en poussière intérieure et inclut la concentration mesurée dans l'information détectée, et
quand la concentration en poussière intérieure est supérieure à une concentration en poussière déterminée, le contrôleur commande à l'orifice de vaporisation de la surface externe (130) et à l'orifice de vaporisation de l'air extérieur (126) d'aspirer de l'air et commande à un orifice de vaporisation de vaporiser de l'air filtré.

5. Porte de douche à air (100) selon la revendication 1, **caractérisée en ce que** :
le troisième détecteur mesure une concentration en poussière extérieure et un ratio de contaminants dans l'air pour chaque composant et inclut un résultat de la mesure dans l'information détectée, et
quand la concentration en poussière extérieure et le ratio de contaminants dans l'air pour chaque composant sont inférieurs à des valeurs préréglées, le contrôleur commande au premier rideau d'air (124) et au deuxième rideau d'air (125) de ne pas fonctionner de façon à ce que l'air extérieur puisse circuler dans l'espace intérieur.
